(19) Europäisches Patentamt
European Patent Office
Office européen des brevets

(11) EP 0 435 152 B1

(12) **EUROPÄISCHE PATENTSCHRIFT**

(45) Veröffentlichungstag und Bekanntmachung des Hinweises auf die Patenterteilung:
**13.11.1996 Patentblatt 1996/46**

(51) Int Cl.[6]: **C12P 41/00**, C12P 17/18, C07D 473/04, C07B 37/10, C07D 473/10

(21) Anmeldenummer: **90124746.0**

(22) Anmeldetag: **19.12.1990**

(54) **Verfahren zur enantioselektiven Darstellung von (omega-1)-Hydroxyalkylxanthinen**

Process for the enantioselective preparation of (omega-1)-hydroxyalkylxanthines

Procédé de préparation énantiosélective de (oméga-1)-hydroxyalkyl-xanthines

(84) Benannte Vertragsstaaten:
**AT BE CH DE DK ES FR GB GR IT LI LU NL SE**

(30) Priorität: **23.12.1989 DE 3942872**

(43) Veröffentlichungstag der Anmeldung:
**03.07.1991 Patentblatt 1991/27**

(73) Patentinhaber: **HOECHST AKTIENGESELLSCHAFT**
**65926 Frankfurt am Main (DE)**

(72) Erfinder:
- **Aretz, Werner, Dr.**
  **W-6240 Königstein/Taunus (DE)**
- **Furrer, Harald, Dr.**
  **W-6238 Hofheim am Taunus (DE)**
- **Gebert, Ulrich, Dr.**
  **W-6246 Schlossborn (DE)**
- **Hinze, Heinz-Joachim**
  **W-6200 Wiesbaden (DE)**

(56) Entgegenhaltungen:
**EP-A- 0 023 032** **WO-A-87/00523**

- **APPLIED ENVIRONMENTAL MICROBIOLOGY Bd. 48, Nr. 2, 1984, Seiten 327 - 331; Davis, P.J.; Yang, S.K.; Robert, V.: 'Microbial models of mammalian metabolism : microbial reduction and oxidation of pentoxifylline.'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Oxalalkylxanthine werden zur Therapie von peripheren, cerebralen und okulären Gefäßerkrankungen eingesetzt. Die bekannteste Substanz aus dieser Gruppe ist das Pentoxifyllin [1-(5-Oxohexyl)-3,7 dimethylxanthin]. Im menschlichen Organismus wird Pentoxifyllin primär zu einem Gemisch enantiomerer Alkohole reduziert. In der WO 87/00523 werden tertiäre Hydroxyalkylxanthine, beispielsweise das 7-Ethoxymethyl-1-(5-hydroxy-5-methylhexyl)-3-methylxanthin, ein Verfahren zu deren Herstellung sowie diese Verbindungen enthaltende, zu Vorbeugung und/oder Behandlung von peripheren und/oder cerebralen Durchblutungsstörungen, geeignete Arzneimittel offenbart.

Weiterhin haben Davis et al. [Appl. Environ. Microbiol. 48, 327 (1984); Xenobiotica 15, 1001(1985)] 13 Kulturen von Mikroorganismen gefunden, die Pentoxifyllin entweder zum Alkohol reduzieren oder die Seitenkette an der Carbonylgruppe spalten und die entsprechenden Carbonsäuren bilden. Ferner beschreiben Davis et al., daß der Stamm Rhodotorula rubra (ATCC 20129) das S-Enantiomer des zu Pentoxifyllin korrespondierenden Alkohols bildet. Mit diesem Stamm wird jedoch innerhalb von 72 Stunden nur eine 56 %ige Umsetzung zum S-Alkohol erreicht bei 40 % präparativer Ausbeute.

Es wurde nun überraschend ein Rhodotorula rubra Stamm gefunden, mit dem in der gleichen Zeit Pentoxifyllin zu ca. 100 % zum S-Alkohol reduziert werden kann bei ca. 62 % präparativer Ausbeute. Darüber hinaus können noch andere Oxoalkylxanthin-Derivate in den korrespondierenden S-Alkohol übergeführt werden. Die entsprechenden S-Alkohole sowie die durch enantioselektive Konfigurationsumkehr erhaltenen R-Alkohole bewirken eine Verstärkung der Gehirndurchblutung.

Die Erfindung betrifft somit ein Verfahren zur enantioselektiven Darstellung von (ω-1)-Hydroxyalkylxanthinen, das dadurch gekennzeichnet ist, daß (ω-1)-Oxoalkylxanthinderivate der allgemeinen Formel I,

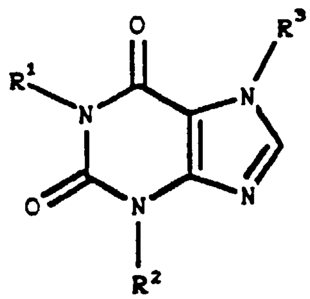

(I)

in der

$R^1$, $R^2$ und $R^3$, die gleich oder verschieden sein können, aus der Gruppe der Substituenten $CH_3$-CO-$(CH_2)_m$- und ($C_1$ bis $C_6$)-Alkyl ausgewählt werden, wobei einer der Substituenten die Gruppe $CH_3$-CO-$(CH_2)_m$ sein muß, m eine ganze Zahl von 2 bis 6 bedeutet, und ($C_1$ bis $C_6$)-Alkyl geradkettig oder verzweigt sein kann, bzw. in der $R^1$ $CH_3$-CO-$(CH_2)_4$-, $R^2$ $CH_3$-und $R^3$ $CH_3$-O-$CH_2$- darstellen, zu den jeweiligen S-(+)-Enantiomeren mit dem Substituenten (S)-$CH_3$-CH(OH) $(CH_2)_m$-, wobei m die obengenannte Bedeutung hat, bzw. zu der Verbindung, in der $R^1$ (S)-$CH_3$-CH(OH)-$(CH_2)_4$-, $R^2$ $CH_3$- und $R^3$ $CH_3$-O-$CH_2$- darstellen, durch Inkubation mit Rhodotorula rubra DSM 5436 reduziert werden.

Die hierbei als Ausgangsstoffe benutzten (ω-1)-Oxoalkylxanthine der Formel I sind, wenn nicht anders angegeben, unter anderem aus den deutschen Auslegeschriften 1 233 405 und 1 235 320 bzw. den deutschen Offenlegungsschriften 23 30 742 und 24 02 908 bekannt oder lassen sich nach bekannten Verfahren leicht herstellen.

Der Stamm Rhodotorula rubra wurde bei der Deutschen Sammlung von Mikroorganismen und Zellkulturen GmbH am 11.07.89 unter der Nummer DSM 5436 nach den Regeln des Budapester Vertrages hinterlegt. Anstelle des hinterlegten Stammes können auch dessen Mutanten und Varianten erfindungsgemäß eingesetzt werden, sofern sie natürlich die Reduktion der Verbindungen der Formel I durchführen können.

Als Substrate können die obengenannten Verbindungen der Formel I verwendet werden. Bevorzugt sind Verbindungen der oben charakterisierten Formel I, in der nur einer der Substituenten $R^1$, $R^2$ und $R^3$ die Gruppe $CH_3$-CO-$(CH_2)_m$- bedeutet, oder in der m die Zahl 2 bis 5 bedeutet und die Alkylgruppe 1 bis 4 C-Atome besitzt, bzw. in der $R^2$ ein Methyl- oder Ethylrest ist. Weiterhin bevorzugt sind Verbindungen der genannten Formel I, in der $R^2$ einen $C_1$ bis $C_4$-Alkylrest und $R^1$ und $R^3$ die Gruppe $CH_3$-CO-$(CH_2)_m$- oder einen $C_1$ bis $C_4$-Alkylrest darstellt, wobei m 2 bis 5 bedeutet. Ebenfalls bevorzugt sind die Verbindungen der genannten Formel I, in der $R^1$ und $R^3$ die Gruppe $CH_3$-CO-$(CH_2)_m$, mit m 3 bis 5, oder $C_1$ bis $C_3$-Alkyl bedeutet und $R^2$ einen Methyl- oder Ethylrest darstellt.

Weitere bevorzugte Verbindungen der charakterisierten Formel I sind solche, in denen m die Zahl 2 bedeutet. Sehr gute Ergebnisse wurden auch mit den Verbindungen der genannten Formel I erzielt, in der $R^1$ und $R^3$ $CH_3$-CO-$(CH_2)_2$- oder einen $C_1$ bis $C_4$-Alkylrest darstellen und $R^2$ ebenfalls ein $C_1$ bis $C_4$-Alkylrest ist.

Insbesondere bevorzugt werden jedoch die Verbindungen der Formel I reduziert, in der

a)

$R^1$ $CH_3$-CO-$(CH_2)_4$-

und

$R^2$ $CH_3$-

und

$R^3$ $CH_3$-$(CH_2)_2$-

oder

b)

$R^1$ und $R^2$ $CH_3$-

und

$R^3$ $CH_3$-CO-$(CH_2)_4$-

oder

c)

$R^1$ $CH_3$-CO-$(CH_2)_4$-

und

$R^2$ $CH_3$-

und

$R^3$ $CH_3$-O-$CH_2$-

oder

d)

$R^1$ $CH_3$-CO-$(CH_2)_4$-

und

$R^2$ und $R^3$ $CH_3$-

oder

e)

$R^1$ und $R^2$ $CH_3(CH_2)_3$-

und

$R^3$ $CH_3CO(CH_2)_2$-

f)

$R^1$ $CH_3$-CO-$(CH_2)_3$-

und

$R^2$ $CH_3$-

und

$R^3$ $CH_3$-$(CH_2)_2$-

oder

g)

$R^1$ $CH_3$-CO-$(CH_2)_5$-

und

$R^2$ und $R^3$ $CH_3$-

bedeutet.

Die genannten Verbindungen können einzeln oder in Gemischen von dem Stamm DSM 5436 verwertet werden.

Die Zugabe des Substrats kann zu einem beliebigen Zeitpunkt während des Wachstums oder der Stationären Phase von Rhodotorula rubra DSM 5436 geschehen. Der Stamm kann im wesentlichen auf allen für das Wachstum geeigneten Nährlösungen aerob, bevorzugt unter Schütteln, bei 25 bis 32°C kultiviert werden. Solche Nährmedien können vom Fachmann schnell ohne erfinderisches Zutun gefunden werden.

Vorteilhaft kann man so vorgehen, daß das Substrat der Formel I der Mikroorganismenkultur nach ca. 5 bis 20stündigem Wachstum in einer Nährlösung, insbesondere nach 8 bis 17 Stunden, zugegeben wird. Die zugegebene Menge des Substrats kann in weiten Bereichen schwanken, bevorzugt sind es jedoch 0,5 bis 10 g/l Nährlösung, insbesondere 0,7 bis 1,2 g/l Nährlösung. Die Nährlösung enthält vorzugsweise Glucose, Sojamehl und Hefeextrakt als Kohlenstoff- bzw. Stickstoffquelle. Es wird über einen Zeitraum von 70 bis 355 Stunden in dem obengenannten Temperaturbereich inkubiert, wobei der Fortgang der Reduktion mittels DC verfolgt werden kann.

Das betreffende Substrat kann auf diese Weise bis zu 100 % zu dem jeweiligen S-(+)-Enantiomeren umgesetzt werden.

Die Erfindung betrifft weiterhin ein Verfahren zur enantioselektiven Herstellung von R-(-)-(ω-1)-Hydroxyalkylxanthinen, bei welchem man in einem ersten Schritt aus der Verbindung der Formel I durch Inkubation mit Rhodotorula rubra DSM 5436 das S-(ω-1)-Hydroxyalkylxanthin herstellt, wonach man in einem weiteren Schritt das jeweilige R-(-)-Enantiomere unter Konfigurationsumkehr erzeugt, das einen (R)-$CH_3$-CH(OH)-$(CH_2)_m$-Rest in einer der Positionen $R^1$, $R^2$ oder $R^3$ der Formel I enthält.

Ein vorteilhaftes Verfahren besteht beispielsweise darin, daß man das mikrobiologisch erhaltene S-(+)-Enantiomer mit einem tertiären Phosphin, bevorzugt Triphenylphosphin, einer Carbonsäure, bevorzugt Benzoesäure und einem Azodicarbonsäruedialkylester, bevorzugt Azodicarbonsäurediethylester, in einem aprotischen Lösungsmittel, bevorzugt Tetrahydrofuran, in das noch als Carbonsäureester vorliegende R-(-)-Enantiomer umwandelt und den Carbonsäureester durch Solvolyse nach bekannten Verfahren, insbesondere durch Methanolyse in Gegenwart von Kaliumcarbonat in die Verbindungen mit einem R(-)-$CH_3$-CH(OH)-$(CH_2)_m$-Rest in einer der Positionen $R^1$, $R^2$ oder $R^3$ der Formel I übergeführt.

Ein weiteres ebenfalls vorteilhaftes Verfahren besteht darin, daß die Hydroxylgruppe am asymmetrischen Kohlenstoffatom der S-(+)-Enantiomeren in einen organischen Sulfonsäureester, bevorzugt einen Methansulfonsäureester oder einen p-Toluolsulfonsäureester umgewandelt wird, der durch nukleophile Substitutionen unter Konfigurationsumkehr entweder direkt in die R-(-)-Enantiomere oder über einen ihrer Carbonsäureester und dessen nachfolgende Solvolyse in die Verbindungen mit einem (R)-$CH_3$-CH(OH)-$(CH_2)_m$-Rest in einer der Positionen $R^1$, $R^2$ oder $R^3$ der Formel I umgewandelt wird. Die Sulfonsäureester werden nach bekannten Methoden durch Reaktion der S-(+)-Hydroxyalkylxanthine mit organischen Sulfonsäurehalogeniden, bevorzugt Methansulfonsäurechlorid und p-Toluolsulfonsäurechlorid, in aprotischen Lösungsmitteln, bevorzugt Pyridin und Dichlormethan, gegebenenfalls in Gegenwart einer Base, wie Triethylamin, hergestellt.

Als geeignete Agentien für die nukleophile Substitution der Sulfonsäureester kommen beispielsweise Alkalisalze aliphatischer Carbonsäuren, bevorzugt Cäsiumpropionat, in aprotischen Lösungsmitteln, wie Dimethylformamid oder Dimethylsulfoxid in Frage. Die Solvolyse der entstandenen Carbonsäureester erfolgt in alkoholischen oder wäßrigen Lösungsmitteln, bevorzugt in Methanol, in Gegenwart von basischen Stoffen, wie z.B. Kaliumcarbonat.

Die Erfindung betrifft ferner die neuen Substanzen 7-Methoxymethyl-3-methyl-1-(5-oxo-hexyl)-xanthin sowie racemisches 1-(5-Hydroxyhexyl)-7-methoxymethyl-3-methylxanthin und die entsprechenden R- und S-Enantiomere.

Die Verbindungen können zur Behandlung von Gefäßerkrankungen eingesetzt werden.

Die im folgenden ausgeführten Beispiele dienen der weiteren Erläuterung der Erfindung.

**Beispiele**

Die Prozentangaben beziehen sich auf das Gewicht, wenn es nicht anders angegeben wird.

**1. Medium und Wachstumsbedingungen**

| Vor- und Hauptkulturmedium | |
|---|---|
| Glucose | 2 % |
| Sojamehl | 0,5 % |
| Hefeextrakt | 0,5 % |
| NaCl | 0,5 % |
| $K_2HPO_4$ | 0,5 % |
| pH 7.0 | |

Als Vorkultur dient ein 2 l Erlenmeyerkolben mit 500 ml Nährlösung, welcher mit einer Abschwemmung vom Schrägröhrchen beimpft wird. Nach einer Inkubationszeit von 72 Stunden bei 28°C und einer Schüttelfrequenz von 250 Upm, wird aus dieser Vorkultur die Hauptkultur mit einem Inokulum von 10 % angeimpft. Die Hauptkultur erfolgt in einem 12 1-Fermenter mit 9 1-Nährlösung bei 28°C, einer Belüftungsrate von 0,1 vvm und einer Rührergeschwindigkeit von 300 Upm.

**Tabelle**

| Bezeichnung des Ausgangsstoffes | Struktur (Formel I) $R^1$ | $R^2$ | $R^3$ | Umsetzungsdauer (Std.) | Umsetzungsgrad (%) | Stereospezifität der Reduktion |
|---|---|---|---|---|---|---|
| 3-Methyl-1-(5-oxohexyl)-7-propyl-xanthin | $CH_3CO-(CH_2)_4$ | $CH_3$ | $(CH_2)_2-CH_3$ | 88 | 100 | > 96 % |
| 1.3-Dimethyl-7-(5-oxohexyl)-xanthin | $H_3C$ | $CH_3$ | $(CH_2)_4-COCH_3$ | 250 | 100 | > 96 % |
| 7-Methoxymethyl-3-methyl-1-(5-oxo-hexyl)-xanthin | $CH_3CO-(CH_2)_4$ | $CH_3$ | $CH_2-O-CH_3$ | 160 | 100 | > 97 % |
| 3.7-Dimethyl-1-(5-oxohexyl)-xanthin (Pentoxifyllin) | $CH_3CO(CH_2)_4$ | $CH_3$ | $CH_3$ | 96 | 100 | > 98 % |
| 1.3-Dibutyl-7-(3-oxobutyl)-xanthin | $C_4H_9$ | $C_4H_9$ | $(CH_2)_2COCH_3$ | 186 | 100 | > 96 % |

**2. Reduktion der Oxoalkylxanthine**

Nach 8 - 17stündigem Wachstum in der Hauptkulturstufe wird der Zellsuspension das sterilfiltrierte Substrat (10 g in 200 ml 50 %igem Ethanol) zugesetzt. Ergebnisse sind beispielhaft in der Tabelle zusammengefaßt. Der Fortgang der Reduktion wird mittels DC verfolgt. Am Ende wird die Kulturlösung abfiltriert und das Kulturfiltrat lyophilisiert.

**3. DC-Analytik**

Je 2 µl Kulturfiltrat werden auf HPTLC-Platten (Kieselgel 60 F 245) aufgetragen und im Fließmittel Chloroform-Ethanol (9:1) entwickelt. Die Auswertung erfolgt bei 270 nm mit Hilfe eines DC-Scanners.

**4. Charakterisierung der reduzierten Endprodukte und ihre Konfigurationsumkehr:**

Die Struktur aller nachstehend beschriebenen Verbindungen wurde durch Elementaranalyse und IR- sowie $^1H$-NMR-Spektren gesichert. Die Bestimmung der absoluten Konfiguration und der Enantiomerenreinheit erfolgte über die Mosher-Ester mit (S-(-)-Methoxy-trifluormethyl-phenylessigsäure ($^1H$- bwz. $^{19}F$-NMR-Spektren) der enantiomeren Hydroxylalkylxanthine. Die Enantiomerenreinheit der alkoholischen Endprodukte wurde auch durch Gaschromatographie nach Derivatisierung mit S-(-)-1-Phenylethylisocyanat ermittelt.

a) S-(+)-1-(5-Hydroxyhexyl)-3,7-dimethylxanthin

Man schlämmt 579 g Lyophilisat (aus der Reduktion von 65 g Pentoxifyllin mit Rhodotorula rubra, DSM 5436) in 3 l Isopropylalkohol auf, fügt 750 g Celite hinzu und filtriert über eine Seitz-Drucknutsche. Den Filterkuchen behandelt man extraktiv mit insgesamt 12 l Isopropylalkohol und engt die vereinigten Filtrate am Rotationsverdampfer bei Wasserstrahlvakuum ein. Den Rückstand nimmt man in Dichlormethan auf, wäscht mit 2n Natronlauge, In Salzsäure, Wasser, trocknet und engt ein. Das Rohprodukt wird durch Chromatographie an einer Mitteldruck-Säule an Kieselgel (Korngröße 20 - 45 µm) und Dichlormethan/Ethanol (Volumenverhältnis 98/2) als Laufmittel gereinigt. Nach Kugelrohrdestillation bei 140 - 150 °C Badtemperatur und 0,3 mbar erhält man 40,8 g an Ausbeute (62,3 % der Theorie).

| | |
|---|---|
| Schmelzpunkt:<br>Enantiomeren-Überschuß (e.e.)<br>$[\alpha]_D^{20}$ | 110 °C<br>> 98 %<br>= 5,7° (c = 6,7, $C_2H_5OH$) |

**Analyse:**

| | | | |
|---|---|---|---|
| ber.: | C 55.7 % | H 7.19 % | N 19.99 % |
| gef.: | C 55.8 % | H 7.34 % | N 20.17 % |

**b) S-(+)-1-(5-Hydroxyhexyl)-3-methyl-7-propyl-xantin**

Analog zu a) wurden 560 g Lyophilisat (aus der Reduktion von 65 g 1-(5-Oxohexyl)-3-methyl-7-propylxanthin mit Rhodotorula rubra, DSM 5436, aufgearbeitet, gereinigt und charakterisiert.

| | |
|---|---|
| Ausbeute: | 32 g (49 % der Theorie)<br>Schmelzpunkt: 81 - 82 °C<br>e.e. > 97 %<br>$[\alpha]_D^{20}$ = + 4,6° (c = 6,6, $C_2H_5OH$) |

**Analyse:**

| | | | |
|---|---|---|---|
| ber.: | C 58.42 % | H 7.84 % | N 18.17 % |
| gef.: | C 58.54 % | H 8.02 % | N 18.15 % |

**c) 1. S-(+)-7-(5-Hydroxyhexyl)-1,3-dimethyl-xanthin 2. 7-(4-Hydroxybutyl)-1,3-dimethylxanthin**

Analog zu a) wurden 430 g Lyophilisat (aus der Reduktion von 30 g 7-(5-Oxohexyl)-1,3-dimethylxanthin mit Rhodotorula rubra DSM 5436) aufgearbeitet, gereinigt und charakterisiert. Es wurden zwei Substanzen erhalten.

| | |
|---|---|
| Zu 1. : Ausbeute: | 6,4 g (21,1 % Ausbeute)<br>Schmelzpunkt: 93°C<br>e.e. > 96 %<br>$[\alpha]_D^{20}$ = + 8,5° (c = 2,3; $C_2H_5OH$) |

**Analyse:**

| | | | |
|---|---|---|---|
| ber.: | C 55,7 % | H 7,19 % | N 19,99 % |
| gef.: | C 55,69 % | H 7,24 % | N 19,65 % |

| | |
|---|---|
| Zu 2.: Ausbeute: | 2,3 g (8,3 % Ausbeute)<br>Schmelzpunkt: 113 - 114°C |

**Analyse:**

| | | | |
|---|---|---|---|
| ber.: | C 52,37 % | H 6,39 % | N 22,21 % |
| gef.: | C 52,35 % | H 6,59 % | N 22,05 % |

**d) S-(+)-1-(5-Hydroxyhexyl)-7-methoxymethyl-3-methylxanthin**

Herstellung des prochiralen 7-Methoxymethyl-3-methyl-1-(5-oxohexyl)-xanthins als Ausgangsstoff:
21,0 g (0,1 mol) 7-Methoxymethyl-3-methylxanthin (hergestellt aus 3-Methylxanthin und Methoxymethylchlorid oder Methoxymethyl-4-toluolsulfonat analog den in DE-OS 35 25 801 ausführlich beschriebenen Verfahrensweisen; $C_8H_{10}N_4O_3$ (MG = 210,2), Schmelzpunkt 251 - 253°C, Analyse: ber. C 45,71 %, H 4,80 %, N 26,66 %; gef. C 45,56 %, H 4,78 %, N 26,60 %) wurden in 500 ml Dimethylformamid gelöst, mit 15,2 g (0,11 mol) Kaliumcarbonat und 14,8 g (0,11 mol) 1-Chlor-5-hexanon versetzt und 18 Stunden bei ca. 110°C kräftig gerührt. Danach wurde das Reaktionsgemisch abgekühlt und unter vermindertem Druck eingedampft, der feste Rückstand zwischen 1 N Natronlauge und Chloroform verteilt und die organische Phase abgetrennt, mit Wasser salzfrei gewaschen, über Natriumsulfat getrocknet und unter vermindertem Druck vom Lösungsmittel befreit.
Umkristallisation aus Essigsäureethylester unter Zusatz von Petrolether in der Siedehitze lieferte 27,1 g (87,9 X der Theorie) analysenreines Keton mit einem Schmelzpunkt von 106 - 107°C.

| $C_{14}H_{20}N_4O_4$ (MG = 308,3) | | | | |
|---|---|---|---|---|
| Analyse: | ber.: | C 54,54 % | H 6,54 % | N 18,17 % |
| | gef.: | C 54,59 % | H 6,57 % | N 17,99 % |

Durch Reduktion von 12,3 g (0,04 mol) dieser Oxohexylverbindung mit 0,76 g (0,02 mol) Natriumborhydrid in 100 ml wasserfreiem Methanol bei Rückflußtemperatur innerhalb von 2 Stunden, Einengen der Mischung unter vermindertem Druck, Aufnahme des Abdampfrückstandes in Chloroform, viermaliges Waschen der Lösung mit wenig Wasser, Trocknen über Natriumsulfat, Abdampfen des Lösungsmittels unter vermindertem Druck und Ausrühren des als Feststoff anfallenden Produktes in Diethylether wurden 8,3 g (66,8 % der Theorie) racemisches 1-(5-Hydroxyhexyl)-7-methoxymethyl-3-methylxanthin mit einem Schmelzpunkt von 63 - 65°C in analysenreiner Form erhalten.

| $C_{14}H_{22}N_4O_4$ (MG = 310,4) | | | | |
|---|---|---|---|---|
| Analyse: | ber.: | C 54,18 % | H 7,15 % | N 18,05 % |
| | gef.: | C 54,07 % | H 7,21 % | N 17,97 % |

240 g Lyophilisat aus der Reduktion von 10 g (0,032 mol) 7-Methoxymethyl-3-methyl-1-(5-oxohexyl)-xanthin mit Rhodotorula rubra, DSM 5436, wurden einer scharfen Gefriertrocknung unterworfen und anschließend in einer Soxhlet-Apparatur 8 Stunden lang mit tert. Butylmethylether extrahiert, wobei sich 8 g (79,5 %) öliges Rohprodukt isolieren ließen. Die Reinigung erfolgte einfachheitshalber durch Chromatographie an einer 43 cm langen Kieselgel-Säule mit einem Durchmesser von 2,8 cm, wobei nach Aufgabe des in Toluol gelösten Rohproduktes lipophile Begleitstoffe mit Toluol eluiert und anschließend der am Kieselgel haftende S-(+)-enantiomere Alkohol zunächst mit reinem tert. Butylmethylether und dann im Gemisch mit Aceton im Volumenverhältnis 7 : 3 ausgewaschen wurde. Das auf diese Weise gewonnene kristalline Reinprodukt enthielt laut Elementaranalyse 1/4 Mol Kristallwasser.

| $C_{14}H_{22}N_4O_4$ x 1/4 $H_2O$ (MG = 314,9) | |
|---|---|
| Ausbeute: | 6,11 g (59,8 % der Theorie) |
| Schmelzpunkt: | 65 - 66°C |
| e.e.: | > 98 % |
| $[\alpha]_D^{20}$ : | + 4,86° (c = 0,5 in Dichlormethan) |

| | | | | |
|---|---|---|---|---|
| Analyse: | ber.: | C 53,41 % | H 7,20 % | N 17,79 % |
| | gef.: | C 53,37 % | H 7,11 % | N 17,83 % |

**e) S-(+)-1,3-Di-n-butyl-7-(3-hydroxybutyl)-xanthin**

Analog zu a) wurden 2,5 g Lyophilisat (aus der Reduktion von 0,25 g 1,3-Di-n-butyl-7-(3-oxobutyl)-xanthin (Schmelzpunkt: 84°C) mit Rhodotorula rubra, DSM 5436) aufgearbeitet, gereinigt und charakterisiert.

| | |
|---|---|
| Ausbeute: | 0,15 g |
| Schmelzpunkt: | 68 - 69°C |
| $[\alpha]_D^{20}$ . | = + 36,3° (c = 3, $C_2H_5OH$) |

Enantiomerenüberschuß (e.e.) > 96 %

| | | | | |
|---|---|---|---|---|
| Analyse: | ber.: | C 60,69 % | H 8,39 % | N 16,65 % |
| | gef.: | C 61,01 % | H 8,59 % | N 16,23 % |

Zum Vergleich wurde racemisches 1,3-Di-n-butyl-7-(3-hydroxybutyl)-xanthin (Schmelzpunkt = 52°C) durch Reduktion von 1,3-Di-n-butyl-7-(3-oxobutyl)-xanthin mit $NaBH_4$ analog zu d) hergestellt.

**f) S-(+)-1-(4-Hydroxypentyl)-3-methyl-7-n-propyl-xanthin**

Analog zu a) wurden 2,7 g Lyophilisat (aus der Reduktion von 0,25 g 1-(4-Oxopentyl)-3-methyl-7-n-propyl-xanthin mit Rhodotorula rubra, DSM 5436) aufgearbeitet, gereinigt und charakterisiert.

| | |
|---|---|
| Ausbeute: | 0,17 g |
| Schmelzpunkt: | 90-91°C |
| $[\alpha]_D^{20}$ | = + 5,8° (c = 3,4, $C_2H_5OH$) |
| Enantiomerenüberschuß (e.e.) | > 98 % |

| | | | | |
|---|---|---|---|---|
| Analyse: | ber.: | C 57,13 % | H 7,53 % | N 19,03 % |

(fortgesetzt)

| | | | | |
|---|---|---|---|---|
| | gef.: | C 57,29 % | H 7,68 % | N 18,70 % |

Zum Vergleich wurde racemisches 1-(4-Hydroxypentyl)-3-methyl-7-n-propyl-xanthin (Schmelzpunkt: 88-89°C) durch Reduktion von 1-(4-Oxopentyl)-3-methyl-7-n-propyl-xanthin mit $NaBH_4$ analog zu d) hergestellt.

**g) S-(+)-1-(6-Hydroxyheptyl)-3,7-dimethyl-xanthin**

Analog Beispiel a) wurden 3 g Lyophilisat (aus der Reduktion von 0,25 g 1-(6-Oxoheptyl)-3,7-dimethyl-xanthin (Schmelzpunkt: 122°C) mit Rhodotorula rubra, DSM 5436) aufgearbeitet, gereinigt und charakterisiert.

| | |
|---|---|
| Ausbeute: | 0,18 g |
| Schmelzpunkt: | 79-81°C |
| $[\alpha]_D^{20}$ | = + 4,2° (c = 3,5, $C_2H_5OH$) |
| e.e. | > 96 % |

| | | | | |
|---|---|---|---|---|
| Analyse: | ber.: | C 57,13 % | H 7,53 % | N 19,03 % |
| | gef.: | C 56,88 % | H 7,93 % | N 18,86 % |

Racemisches 1-(6-Hydroxyheptyl)-3,7-dimethylxanthin (Schmelzpunkt: 76-77°C) wurde zum Vergleich durch Reduktion von 1-(6-Oxoheptyl)-3,7-dimethyl-xanthin mit $NaBH_4$ analog zu d) hergestellt.

**h) R-(-)-1-(5-Hydroxyhexyl)-3,7-dimethylxanthin**

Zur Lösung von 29,8 g S-(+)-1-(5-Hydroxyhexyl)-3,7-dimethylxanthin, 17,8 g Benzoesäure und 53,1 g Triphenylphosphin in 120 ml absolutem Tetrahydrofuran tropft man bei Raumtemperatur während 15 min 36 g Azodicarbonsäurediethylester in 120 ml absolutem Tetrahydrofuran. Nach 8-stündigem Rühren bei Raumtemperatur wird bei vermindertem Druck eingeengt und der Rückstand (150 g) auf einer Mitteldrucksäule an Kieselgel (20-45 µm Korngröße und 6 nm Porengröße) mit Dichlormethan/Methanol im Volumenverhältnis 98:2 als Laufmittel gereinigt.

Es fallen 78 g rohes R-(-)-1-(5-Benzoyloxyhexyl)-3,7-dimethylxanthin an, die als Lösung in 300 ml Methanol 48 Stunden bei Raumtemperatur zusammen mit 5 g Kaliumcarbonat gerührt werden. Nach Einengen bei vermindertem Druck wird mit Dichlormethan aufgenommen, abfiltriert und die Lösung wie oben, aber mit Dichlormethan/Methanol im Volumenverhältnis 95:5 chromatographiert. Die dünnschichtchromatographisch sauberen Fraktionen werden über Kugelrohr bei 150-155°C Badtemperatur und 0,3 mbar destilliert. Man erhält 21,2 g an Ausbeute (71,1 % der Theorie).

| | |
|---|---|
| Schmelzpunkt: | 110°C |
| $[\alpha]_D^{20}$ | = -5,6° (c = 6,7, $C_2H_5OH$) |
| Enantiomerenüberschuß (e.e.) | > 98 % |

| | | | | |
|---|---|---|---|---|
| Analyse: | ber.: | C 55,7 % | H 7,19 % | N 19,99 % |
| | gef.: | C 55,63 % | H 7,26 % | N 19,91 % |

**i) R-(-)-1-(5-Hydroxyhexyl)-3-methyl-7-propyl-xanthin)**

Die Darstellung und nachfolgende Reinigung erfolgen analog zu h) aus 19 g S-(+)-1-(5-Hydroxyhexyl)-3-methyl-7-propyl-xanthin

| | |
|---|---|
| Ausbeute: | 14,7 g (77,4 % der Theorie) |
| Schmelzpunkt: | 81-82°C |
| $[\alpha]_D^{20}$ | = -4,5° (c = 6,3, $C_2H_5OH$) |

(fortgesetzt)

| | |
|---|---|
| e.e. | > 98 % |

| | | | | |
|---|---|---|---|---|
| Analyse: | ber.:<br>gef.: | C 58,42 %<br>C 58,36 % | H 7,84 %<br>H 8,08 % | N 18,17 %<br>N 18,22 % |

**j) R-(-)-7-(5-Hydroxyhexyl)-1,3-dimethyl-xanthin**

Umsetzung und nachfolgende Reinigung erfolgen analog zu h) ausgehend von 3,4 g S-(+)-7-(5-Hydroxyhexyl)-1,3-dimethyl-xanthin.

| | |
|---|---|
| Ausbeute: | 2,4 g (70,4 % der Theorie) |
| Schmelzpunkt | 93°C |
| $[\alpha]_D^{20}$ | = -8,4° (c = 2,2, $C_2H_5OH$) |
| e.e. | > 96 % |

| | | | | |
|---|---|---|---|---|
| Analyse: | ber.:<br>gef.: | C 55,7 %<br>C 55,73 % | H 7,19 %<br>H 7,17 % | N 19,99 %.<br>N 19,73 %. |

**k) R-(-)-1,3-Di-n-butyl-7-(3-hydroxybutyl)-xanthin**

Die Umsetzung, nachfolgende Reinigung und Charakterisierung erfolgten analog Beispiel h ausgehend von 6,8 g S-(+)-1,3-Di-n-butyl-7-(3-hydroxybutyl)-xanthin.

| | |
|---|---|
| Ausbeute: | 3,8 g |
| Schmelzpunkt: | 67-68°C |
| $[\alpha]_D^{20}$ | = -34,6° (c = 2, $C_2H_5OH$) |
| e.e. | > 96 % |

| | | | | |
|---|---|---|---|---|
| Analyse: | ber.:<br>gef.: | C 60,69 %<br>C 60,30 % | H 8,39 %<br>H 8,57 % | N 16,65 %<br>N 16,64 % |

**1) R-(-)-1-(4-Hydroxypentyl)-3-methyl-7-n-propyl-xanthin**

Die Umsetzung, nachfolgende Reinigung und Charakterisierung erfolgten analog Beispiel h ausgehend von 3,3 g S-(+)-1-(4-Hydroxypentyl)-3-methyl-7-n-propyl-xanthin.

| | |
|---|---|
| Ausbeute: | 2,2 g |
| Schmelzpunkt | 90°C |
| $[\alpha]_D^{20}$ | = -6,2° (c = 0,8, $C_2H_5OH$) |
| e.e. | > 97 % |

| | | | | |
|---|---|---|---|---|
| Analyse: | ber.:<br>gef.: | C 57,13 %<br>C 56,78 % | H 7,53 %<br>H 7,46 % | N 19,03 %.<br>N 18,83 %. |

**m) R-(-)-1-(6-Hydroxyheptyl)-3,7-dimethylxanthin**

Die Umsetzung, nachfolgende Reinigung und Charakterisierung erfolgten analog Beispiel h ausgehend von 11,6 g S-(+)-1-(6-Hydroxyheptyl)-3,7-dimethylxanthin.

| | |
|---|---|
| Ausbeute: | 6,2 g |
| Schmelzpunkt: | 82-84°C |
| $[\alpha]_D^{20}$ | = -4,8° (c = 1,6, $C_2H_5OH$) |
| e.e. | > 96 % |

| | | | | |
|---|---|---|---|---|
| Analyse: | ber.: | C 57,13 % | H 7,53 % | N 19,03 % |
| | gef.: | C 56,77 % | H 7,78 % | N 19,01 % |

**Patentansprüche**

**Patentansprüche für folgende Vertragsstaaten : AT, BE, CH, DE, DK, FR, GB, IT, LI, LU, NL, SE**

**1.** Verfahren zur enantioselektiven Herstellung von S-(ω-1)-Hydroxyalkylxanthinen, dadurch gekennzeichnet, daß die Verbindung der allgemeinen Formel I,

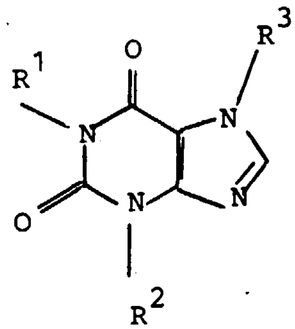

I

in der

$R^1$, $R^2$ und $R^3$, die gleich oder verschieden sein können, aus der Gruppe der Substituenten $CH_3$-CO-$(CH_2)_m$- und ($C_1$ bis $C_6$)-Alkyl ausgewählt werden, wobei einer der Substituenten die Gruppe $CH_3$-CO-$(CH_2)_m$ sein muß, m eine ganze Zahl von 2 bis 6 ist und ($C_1$ bis $C_6$)-Alkyl geradkettig oder verzweigt sein kann oder in der
$R^1$ $CH_3$-CO-$(CH_2)_4$-, $R^2$ $CH_3$- und $R^3$ $CH_3$-O-$CH_2$- darstellen, mit Rhodotorula rubra DSM 5436 inkubiert wird.

**2.** Verfahren nach Anspruch 1, dadurch gekennzeichnet, daß als Verbindung der allgemeinen Formel I die Verbindung, in der nur einer der Substituenten $R^1$, $R^2$ und $R^3$ die Gruppe $CH_3$-CO-$(CH_2)_m$ bedeutet, eingesetzt wird.

**3.** Verfahren nach Anspruch 1, dadurch gekennzeichnet, daß als Verbindung der allgemeinen Formel I die Verbindung, in der

a)

$R^1$ $CH_3$-CO-$(CH_2)_4$-

und

$R^2$ $CH_3$-

und

$R^3$ $CH_3$-$(CH_2)_2$-

oder

b)

$R^1$ und $R^2$ $CH_3$-

und

$R^3$ $CH_3$-$CO(CH_2)_4$-

oder

c)

$R^1$ $CH_3$-$CO(CH_2)_4$-

und

$R^2$ $CH_3$-

und

$R^3$ $CH_3$-O-$CH_2$-

oder

d)

$R^1$ $CH_3$-CO-$(CH_2)_4$-

und

$R^2$ und $R^3$ $CH_3$-

oder

e)

$R^1$ und $R^2$ $CH_3$-$(CH_2)_3$-

und

$R^3$ $CH_3CO(CH_2)_2$-

f)

$R^1$ $CH_3$-CO-$(CH_2)_3$-

und

$R^2$ $CH_3$-

und

$R^3$ $CH_3$-$(CH_2)_2$-

oder

g)

$R^1$ $CH_3$-CO-$(CH_2)_5$-

und

$R^2$ und $R^3$ $CH_3$-

eingesetzt wird.

**4.** Verfahren nach einem oder mehreren der Ansprüche 1 bis 3, dadurch gekennzeichnet, daß bei 25 bis 32°C inkubiert wird.

**5.** Verfahren zur enantioselektiven Herstellung von R-(-)-(ω-1)-Hydroxyalkylxanthinen, dadurch gekennzeichnet, daß man in einem ersten Schritt ein S-(+)-(ω-1)-Hydroxyalkylxanthin nach einem Verfahren gemäß einem der Ansprüche 1 bis 4 herstellt, wonach man in einem weiteren Schritt das genannte S-(+)-(ω-1)-Hydroxyalkylxanthin mit einem tertiären Phosphin, einer Carbonsäure und einem Azodicarbonsäuredialkylester in einem aprotischen Lösungsmittel zur Reaktion bringt und das Reaktionsprodukt durch Solvolyse in eine Verbindung der Formel I gemäß Anspruch 1 überführt, in der einer der Substituenten $R^1$, $R^2$ oder $R^3$ R-(-)-$CH_3$-CH(OH)-$(CH_2)_m$ bedeutet.

**6.** Verfahren nach Anspruch 5, dadurch gekennzeichnet, daß das tertiäre Phosphin Triphenylphosphin, die Carbonsäure Benzoesäure, der Azodicarbonsäuredialkylester Azodicarbonsäurediethylester, das aprotische Lösungsmittel Tetrahydrofuran ist, und die Solvolyse in Form einer Methanolyse in Gegenwart von Kaliumcarbonat durchgeführt wird.

**7.** Verfahren zur enantioselektiven Herstellung von R-(-)-(ω-1)-Hydroxyalkylxanthinen, dadurch gekennzeichnet, daß man in einem ersten Schritt ein S-(+)-(ω-1)-Hydroxyalkylxanthin nach einem Verfahren gemäß einem der Ansprüche 1 bis 4 herstellt, wonach man in einem weiteren Schritt

a) das genannte S-(-)-(ω-1)-Hydroxyalkylxanthin mit organischen Sulfonsäurehalogeniden in aprotischen Lösungsmitteln zur Reaktion bringt,

b) mit Alkalisalzen aliphatischer Carbonsäuren in aprotischen Lösungsmitteln umsetzt und

c) durch Solvolyse in alkoholischen oder wäßrigen Lösungsmitteln in Gegenwart von basischen Stoffen in die Verbindung der Formel I nach Anspruch 1, in der einer der Substituenten $R^1$, $R^2$ oder $R^3$ R-(-)$CH_3$-CH(OH)-

$(CH_2)_m$ bedeutet, überführt.

**8.** Verfahren nach Anspruch 7, dadurch gekennzeichnet, daß gemäß a) das organische Sulfonsäurehalogenid, Methansulfonsäurechlorid oder p-Toluolsulfonsäurechlorid, das aprotische Lösungsmittel Pyridin oder Dichlormethan und daß gemäß b) das Alkalisalz aliphatischer Carbonäsuren Cäsiumpropionat, das aprotische Lösungsmittel Dimethylformamid oder Dimethylsulfoxid und das gemäß c) das Lösungsmittel Methanol und der basische Stoff Kaliumcarbonat ist.

**9.** Rhodotorula rubra DSM 5436 sowie deren Mutanten und Varianten, sofern mit ihnen das Verfahren nach dem Anspruch 1 durchgeführt werden kann.

**10.** 7-Methoxymethyl-3-methyl-1-(5-oxohexyl)-xanthin.

**11.** Racemisches 1-(5-Hydroxyhexyl)-7-methoxymethyl-3-methylxanthin sowie dessen R- und S-Enantiomere.

**Patentansprüche für folgende Vertragsstaaten : ES, GR**

**1.** Verfahren zur enantioselektiven Herstellung von S-(ω-1)-Hydroxyalkylxanthinen, dadurch gekennzeichnet, daß die Verbindung der allgemeinen Formel I,

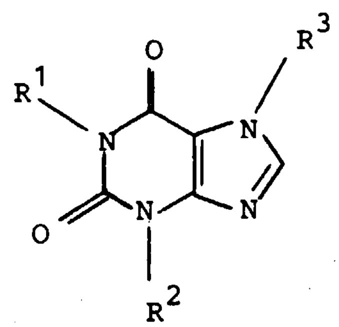

I

in der

$R^1$, $R^2$ und $R^3$, die gleich oder verschieden sein können, aus der Gruppe der Substituenten $CH_3$-CO-$(CH_2)_m$- und ($C_1$ bis $C_6$)-Alkyl ausgewählt werden, wobei einer der Substituenten die Gruppe $CH_3$-CO-$(CH_2)_m$ sein muß, m eine ganze Zahl von 2 bis 6 ist und ($C_1$ bis $C_6$)-Alkyl geradkettig oder verzweigt sein kann oder in der
$R^1$ $CH_3$-CO-$(CH_2)_4$-, $R^2$ $CH_3$- und $R^3$ $CH_3$-O-$CH_2$- darstellen, mit Rhodotorula rubra DSM 5436 inkubiert wird.

**2.** Verfahren nach Anspruch 1, dadurch gekennzeichnet, daß als Verbindung der allgemeinen Formel I die Verbindung, in der nur einer der Substituenten $R^1$, $R^2$ und $R^3$ die Gruppe $CH_3$-CO-$(CH_2)_m$ bedeutet, eingesetzt wird.

**3.** Verfahren nach Anspruch 1, dadurch gekennzeichnet, daß als Verbindung der allgemeinen Formel I die Verbindung, in der

a)

$R^1$ $CH_3$-CO-$(CH_2)_4$-

und

$R^2$ $CH_3$-

und

$R^3$ $CH_3$-$(CH_2)_2$-

oder

b)

$R^1$ und $R^2$ $CH_3$-

und

$R^3$ $CH_3$-$CO(CH_2)_4$-

oder

c)

$R^1$ $CH_3$-$CO(CH_2)_4$-

und

$R^2$ $CH_3$-

und

$R^3$ $CH_3$-O-$CH_2$-

oder

d)

$R^1$ $CH_3$-CO-$(CH_2)_4$-

und

$R^2$ und $R^3$ $CH_3$-

oder

e)

$R^1$ und $R^2$ $CH_3$-$(CH_2)_3$-

und

$R^3$ $CH_3CO(CH_2)_2$-

f)

$R^1$ $CH_3$-CO-$(CH_2)_3$-

und

$R^2$ $CH_3$-

und

$R^3$ $CH_3$-$(CH_2)_2$-

oder

g)

$R^1$ $CH_3$-CO-$(CH_2)_5$-

und

$R^2$ und $R^3$ $CH_3$-

eingesetzt wird.

**4.** Verfahren nach einem oder mehreren der Ansprüche 1 bis 3, dadurch gekennzeichnet, daß bei 25 bis 32°C inkubiert wird.

**5.** Verfahren zur enantioselektiven Herstellung von R-(-)-(ω-1)-Hydroxyalkylxanthinen, dadurch gekennzeichnet, daß man in einem ersten Schritt ein S-(+)-(ω-1)-Hydroxyalkylxanthin nach einem Verfahren gemäß einem der Ansprüche 1 bis 4 herstellt, wonach man in einem weiteren Schritt das genannte S-(+)-(ω-1)-Hydroxyalkylxanthin mit einem tertiären Phosphin, einer Carbonsäure und einem Azodicarbonsäuredialkylester in einem aprotischen Lösungsmittel zur Reaktion bringt und das Reaktionsprodukt durch Solvolyse in eine Verbindung der Formel I gemäß Anspruch 1 überführt, in der einer der Substituenten $R^1$, $R^2$ oder $R^3$ R-(-)-$CH_3$-CH(OH)-$(CH_2)_m$ bedeutet.

**6.** Verfahren nach Anspruch 5, dadurch gekennzeichnet, daß das tertiäre Phosphin Triphenylphosphin, die Carbonsäure Benzoesäure, der Azodicarbonsäuredialkylester Azodicarbonsäurediethylester, das aprotische Lösungsmittel Tetrahydrofuran ist, und die Solvolyse in Form einer Methanolyse in Gegenwart von Kaliumcarbonat durchgeführt wird.

**7.** Verfahren zur enantioselektiven Herstellung von R-(-)-(ω-1)-Hydroxyalkylxanthinen, dadurch gekennzeichnet, daß man in einem ersten Schritt ein S-(+)-(ω-1)-Hydroxyalkylxanthin nach einem Verfahren gemäß einem der Ansprüche 1 bis 4 herstellt, wonach man in einem weiteren Schritt

a) das genannte S-(-)-(ω-1)-Hydroxyalkylxanthin mit organischen Sulfonsäurehalogeniden in aprotischen Lösungsmitteln zur Reaktion bringt,

b) mit Alkalisalzen aliphatischer Carbonsäuren in aprotischen Lösungsmitteln umsetzt und

c) durch Solvolyse in alkoholischen oder wäßrigen Lösungsmitteln in Gegenwart von basischen Stoffen in die Verbindung der Formel I nach Anspruch 1, in der einer der Substituenten $R^1$, $R^2$ oder $R^3$ R-(-)$CH_3$-CH(OH)-$(CH_2)_m$ bedeutet, überführt.

**8.** Verfahren nach Anspruch 7, dadurch gekennzeichnet, daß gemäß a) das organische Sulfonsäurehalogenid, Methansulfonsäurechlorid oder p-Toluolsulfonsäurechlorid, das aprotische Lösungsmittel Pyridin oder Dichlormethan und daß gemäß b) das Alkalisalz aliphatischer Carbonäsuren Cäsiumpropionat, das aprotische Lösungsmittel Dimethylformamid oder Dimethylsulfoxid und das gemäß c) das Lösungsmittel Methanol und der basische Stoff Kaliumcarbonat ist.

**9.** Rhodotorula rubra DSM 5436 sowie deren Mutanten und Varianten, sofern mit ihnen das Verfahren nach dem Anspruch 1 durchgeführt werden kann.

## Claims

**Claims for the following Contracting States : AT, BE, CH, LI, DE, DK, FR, GB, IT, LU, NL, SE**

**1.** A process for the enantioselective preparation of S-(ω-1)-hydroxyalkylxanthines, which comprises incubating the compound of the formula I

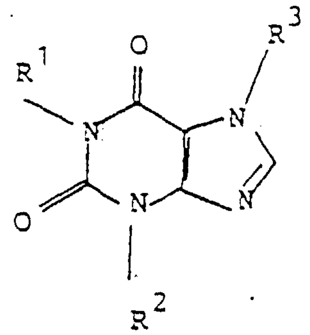

I

in which

$R^1$, $R^2$ and $R^3$, which can be identical or different, are chosen from the group comprising the substituents $CH_3$-CO-$(CH_2)_m$- and ($C_1$ to $C_6$)-alkyl, where one of the substituents must be the group $CH_3$-CO-$(CH_2)_m$, m is an integer from 2 to 6, and ($C_1$ to $C_6$)-alkyl can be straight-chain or branched, or
in which
$R^1$ is $CH_3$-CO-$(CH_2)_4$-, $R^2$ is $CH_3$- and $R^3$ is $CH_3$-O-$CH_2$-, with Rhodotorula rubra DSM 5436.

**2.** The process as claimed in claim 1, wherein the compound in which only one of the substituents $R^1$, $R^2$ and $R^3$ is the group $CH_3$-CO-$(CH_2)_m$ is employed as the compound of the formula I.

**3.** The process as claimed in claim 1, wherein the compound in which

a)

$R^1$ is $CH_3$-CO-$(CH_2)_4$-

and

$R^2$ is $CH_3$-

and

$R^3$ is $CH_3$-$(CH_2)_2$-

or

b)

$R^1$ and $R^2$ are $CH_3$-

and

$R^3$ is $CH_3$-CO-$(CH_2)_4$-

or

c)

$R^1$ is $CH_3$-CO-$(CH_2)_4$-

and

$R^2$ is $CH_3$-

and

$R^3$ is $CH_3$-O-$CH_2$-

or

d)

$R^1$ is $CH_3$-CO-$(CH_2)_4$-

and

$R^2$ and $R^3$ are $CH_3$-

or

e)

$R^1$ and $R^2$ are $CH_3(CH_2)_3$-

and

$R^3$ is $CH_3CO(CH_2)_2$-

f)

$R^1$ is $CH_3$-CO-$(CH_2)_3$-

and

$R^2$ is $CH_3$-

and

$R^3$ is $CH_3$-$(CH_2)_2$-

or

g)

$R^1$ is $CH_3$-CO-$(CH_2)_5$-

and

$R^2$ and $R^3$ are $CH_3$-

is employed as the compound of the formula I.

**4.** The process as claimed in one or more of claims 1 to 3, wherein the incubation is carried out at 25 to 32°C.

**5.** A process for the enantioselective preparation of R-(-)-(ω-1)-hydroxyalkylxanthines, which comprises, in a first step, preparing an S-(+)-(ω-1)-hydroxyalkylxanthine by a process as claimed in any one of claims 1 to 4, after which, in a further step, said S-(+)-(ω-1)-hydroxyalkylxanthine is reacted in an aprotic solvent with a tertiary phosphine, a carboxylic acid and a dialkyl azodicarboxylate and the reaction product is converted by solvolysis into a compound of the formula I as claimed in claim 1 in which one of the substituents $R^1$, $R^2$ or $R^3$ is R-(-)-$CH_3$-CH (OH)-$(CH_2)_m$.

**6.** The process as claimed in claim 5, wherein the tertiary phosphine is triphenylphosphine, the carboxylic acid is benzoic acid, the dialkyl azodicarboxylate is diethyl azodicarboxylate, the aprotic solvent is tetrahydrofuran, and the solvolysis is carried out in the form of a methanolysis in the presence of potassium carbonate.

**7.** A process for the enantioselective preparation of R-(-)-(ω-1)-hydroxyalkylxanthines, which comprises, in a first step, preparing an S-(+)-(ω-1)-hydroxyalkylxanthine by a process as claimed in any one of claims 1 to 4, after which, in a further step,

a) said S-(-)-(ω-1)-hydroxyalkylxanthine is reacted with organic sulfonyl halides in aprotic solvents,

b) reacted with alkali metal salts of aliphatic carboxylic acids in aprotic solvents and

c) converted into the compound of the formula I as claimed in claim 1 in which one of the substituents $R^1$, $R^2$ or $R^3$ is R-(-)$CH_3$-CH(OH)-$(CH_2)_m$ by solvolysis in alcoholic or aqueous solvents in the presence of basic substances.

**8.** The process as claimed in claim 7, wherein the organic sulfonyl halide as in a) is methanesulfonyl chloride or p-toluenesulfonyl chloride, the aprotic solvent is pyridine or dichloromethane and the alkali metal salt of aliphatic carboxylic acids as in b) is cesium propionate, the aprotic solvent is dimethylformamide or dimethyl sulfoxide and the solvent as in c) is merhanol and the basic substance is potassium carbonate.

**9.** Rhodotorula rubra DSM 5436 and its mutants and variants, if the process as claimed in claim 1 can be carried out using them.

**10.** 7-Methoxymethyl-3-methyl-1-(5-oxohexyl)xanthine.

**11.** Racemic 1-(5-hydroxyhexyl)-7-methoxymethyl-3-methyl-xanthine and its R- and S-enantiomers.

**Claims for the following Contracting States : ES, GR**

**1.** A process for the enantioselective preparation of S-(ω-1)-hydroxyalkylxanthines, which comprises incubating the compound of the formula I

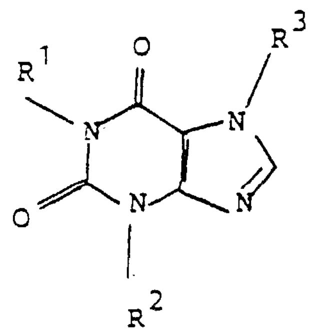

I

in which

$R^1$, $R^2$ and $R^3$, which can be identical or different, are chosen from the group comprising the substituents $CH_3$-CO-$(CH_2)_m$- and ($C_1$ to $C_6$)-alkyl, where one of the substituents must be the group $CH_3$-CO-$(CH_2)_m$, m is an integer from 2 to 6, and ($C_1$ to $C_6$)-alkyl can be straight-chain or branched, or
in which
$R^1$ is $CH_3$-CO-(-$CH_2)_4$-, $R^2$ is $CH_3$- and $R^3$ is $CH_3$-O-$CH_2$-, with Rhodotorula rubra DSM 5436.

**2.** The process as claimed in claim 1, wherein the compound in which only one of the substituents $R^1$, $R^2$ and $R^3$ is the group $CH_3$-CO-$(CH_2)_m$ is employed as the compound of the formula I.

**3.** The process as claimed in claim 1, wherein the compound in which

a)

$R^1$ is $CH_3$-CO-$(CH_2)_4$-

and

$R^2$ is $CH_3$-

and

$R^3$ is $CH_3$-$(CH_2)_2$-

or

b)

$R^1$ and $R^2$ are $CH_3$-

and

$R^3$ is $CH_3$-CO-$(CH_2)_4$-

or

c)

$R^1$ is $CH_3$-CO-$(CH_2)_4$-

and

$R^2$ is $CH_3$-

and

$R^3$ is $CH_3$-O-$CH_2$-

or

d)

$R^1$ is $CH_3$-CO-$(CH_2)_4$-

and

$R^2$ and $R^3$ are $CH_3$-

or

e)

$R^1$ and $R^2$ are $CH_3(CH_2)_3$-

and

$R^3$ is $CH_3CO(CH_2)_2$-

f)

$R^1$ is $CH_3$-CO-$(CH_2)_3$-

and

$R^2$ is $CH_3$-

and

$R^3$ is $CH_3$-$(CH_2)_2$-

or

g)

$R^1$ is $CH_3$-CO-$(CH_2)_5$-

and

$R^2$ and $R^3$ are $CH_3$-

is employed as the compound of the formula I.

**4.** The process as claimed in one or more of claims 1 to 3, wherein the incubation is carried out at 25 to 32°C.

**5.** A process for the enantioselective preparation of R-(-)-(ω-1)-hydroxyalkylxanthines, which comprises, in a first step, preparing an S-(+)-(ω-1)-hydroxyalkylxanthine by a process as claimed in any one of claims 1 to 4, after which, in a further step, said S-(+)-(ω-1)-hydroxyalkylxanthine is reacted in an aprotic solvent with a tertiary phosphine, a carboxylic acid and a dialkyl azodicarboxylate and the reaction product is converted by solvolysis into a compound of the formula I as claimed in claim 1 in which one of the substituents $R^1$, $R^2$ or $R^3$ is R-(-)-$CH_3$-CH(OH)-$(CH_2)_m$.

**6.** The process as claimed in claim 5, wherein the tertiary phosphine is triphenylphosphine, the carboxylic acid is benzoic acid, the dialkyl azodicarboxylate is diethyl azodicarboxylate, the aprotic solvent is tetrahydrofuran, and the solvolysis is carried out in the form of a methanolysis in the presence of potassium carbonate.

**7.** A process for the enantioselective preparation of R-(-)-(ω-1)-hydroxyalkylxanthines, which comprises, in a first step, preparing an S-(+)-(ω-1)-hydroxyalkylxanthine by a process as claimed in any one of claims 1 to 4, after which, in a further step,

a) said S-(-)-(ω-1)-hydroxyalkylxanthine is reacted with organic sulfonyl halides in aprotic solvents,

b) reacted with alkali metal salts of aliphatic carboxylic acids in aprotic solvents and

c) converted into the compound of the formula I as claimed in claim 1 in which one of the substituents $R^1$, $R^2$ or $R^3$ is R-(-)$CH_3$-CH(OH)-$(CH_2)_m$ by solvolysis in alcoholic or aqueous solvents in the presence of basic substances.

**8.** The process as claimed in claim 7, wherein the organic sulfonyl halide as in a) is methanesulfonyl chloride or p-toluenesulfonyl chloride, the aprotic solvent is pyridine or dichloromethane and the alkali metal salt of aliphatic carboxylic acids as in b) is cesium propionate, the aprotic solvent is dimethylformamide or dimethyl sulfoxide and the solvent as in c) is methanol and the basic substance is potassium carbonate.

**9.** Rhodotorula rubra DSM 5436 and its mutants and variants, if the process as claimed in claim 1 can be carried out using them.

## Revendications

### Revendications pour les Etats contractants suivants : AT, BE, CH, LI, DE, DK, FR, GB, IT, LU, NL, SE

**1.** Procédé pour la préparation énantiosélective de S-(ω-1)-hydroxyalkylxanthines, caractérisé en ce que l'on met à incuber avec *Rhodotorula rubra* DSM 5436 un composé de formule générale I

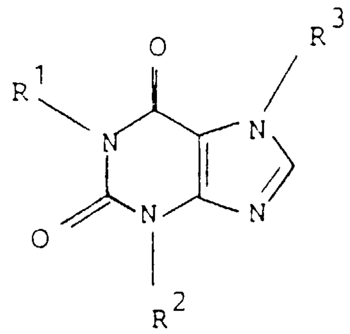

I

dans laquelle $R^1$, $R^2$ et $R^3$, qui peuvent être identiques ou différents, sont choisis dans le groupe constitué par les substituants $CH_3$-CO-$(CH_2)_m$- et alkyle en $C_1$-$C_6$, l'un des substituants devant être le groupe $CH_3$-CO-$(CH_2)_m$, m représentant un nombre entier allant de 2 à 6, et le groupe alkyle en $C_1$-$C_6$ pouvant être à chaîne droite ou ramifiée, ou dans laquelle
$R^1$ représente le groupe $CH_3$-CO-$(CH_2)_4$-, $R^2$ représente le groupe $CH_3$- et $R^3$ représente le groupe $CH_3$-O-$CH_2$-.

**2.** Procédé selon la revendication 1, caractérisé en ce que l'on utilise comme composé de formule générale I le composé dans lequel un seul des substituants $R^1$, $R^2$ et $R^3$ représente le groupe $CH_3$-CO-$(CH_2)_m$.

**3.** Procédé selon la revendication 1, caractérisé en ce que l'on utilise comme composé de formule générale I le composé dans lequel

a)

$R^1$ représente $CH_3$-CO-$(CH_2)_4$-,
$R^2$ représente $CH_3$- et
$R^3$ représente $CH_3$-$(CH_2)_2$- ou

b)

$R^1$ et $R^2$ représentent $CH_3$ - et
$R^3$ représente $CH_3$-CO-$(CH_2)_4$- ou

c)

$R^1$ représente $CH_3$-CO-$(CH_2)_4$-,
$R^2$ représente $CH_3$- et
$R^3$ représente $CH_3$-O-$CH_2$- ou

d)

$R^1$ représente $CH_3$-CO-$(CH_2)_4$- et
$R^2$ et $R^3$ représentent $CH_3$-, ou

e)

$R^1$ et $R^2$ représentent $CH_3$-$(CH_2)_3$- et
$R^3$ représente $CH_3CO(CH_2)_2$- ou

f)

$R^1$ représente $CH_3$-CO-$(CH_2)_3$-,
$R^2$ représente $CH_3$- et
$R^3$ représente $CH_3$-$(CH_2)_2$- ou

g)

$R^1$ représente $CH_3$-CO-$(CH_2)_5$- et
$R^2$ et $R^3$ représentent $CH_3$-.

**4.** Procédé selon une ou plusieurs des revendications 1 à 3, caractérisé en ce que l'on effectue l'incubation à 25-32°C.

**5.** Procédé pour la préparation énantiosélective de R-(-)-(ω-1)-hydroxyalkylxanthines, caractérisé en ce que, dans une première étape, on prépare une S-(+)-(ω-1)-hydroxyalkylxanthine, selon un procédé conforme à l'une des revendications 1 à 4, à la suite de quoi, dans une seconde étape, on fait réagir ladite S-(+)-(ω-1)-hydroxyalkylxanthine avec une phosphine tertiaire, un acide carboxylique et un azodicarboxylate de dialkyle, dans un solvant aprotique, et on convertit le produit de réaction, par solvolyse, en un composé de formule I selon la revendication 1, dans lequel l'un des substituants $R^1$, $R^2$ et $R^3$ représente le groupe R-(-)-$CH_3$-CH(OH)-$(CH_2)_m$.

**6.** Procédé selon la revendication 5, caractérisé en ce que la phosphine tertiaire est la triphénylphosphine, l'acide carboxylique est l'acide benzoïque, l'azodicarboxylate de dialkyle est l'azodicarboxylate de diéthyle, le solvant aprotique est le tétrahydrofuranne et la solvolyse est effectuée sous forme d'une méthanolyse en présence de carbonate de potassium.

**7.** Procédé pour la préparation énantiosélective de R-(-)-(ω-1)-hydroxyalkylxanthines, caractérisé en ce que, dans une première étape, on prépare une S-(+)-(ω-1)-hydroxyalkylxanthine, selon un procédé conforme à l'une des revendications 1 à 4, à la suite de quoi, dans une seconde étape

a) on fait réagir ladite S-(-)-(ω-1)-hydroxyalkylxanthine avec des halogénures de sulfonyle organiques, dans des solvants aprotiques,
b) on fait réagir le produit avec des sels alcalins d'acides carboxyliques aliphatiques, dans des solvants aprotiques, et
c) on convertit le produit par solvolyse dans des solvants aqueux ou alcooliques, en présence de substances basiques, en un composé de formule I selon la revendication 1, dans lequel l'un des substituants $R^1$, $R^2$ et $R^3$ représente le groupe R-(-)-$CH_3$-CH(OH)-$(CH_2)_m$.

**8.** Procédé selon la revendication 7, caractérisé en ce que selon a) l'halogénure de sulfonyle organique est le chlorure de méthanesulfonyle ou le chlorure de p-toluènesulfonyle, le solvant aprotique est la pyridine ou le dichlorométhane, et en ce que selon b) le sel alcalin d'acides carboxyliques aliphatiques est le propionate de césium, le solvant aprotique est le diméthylformamide ou le diméthylsulfoxyde et en ce que selon c) le solvant est le méthanol et la substance basique est le carbonate de potassium.

**9.** *Rhodotorula rubra* DSM 5436 ainsi que ses mutants et variants, dans la mesure où le procédé selon la revendication 1 peut être mis en oeuvre avec ces derniers.

**10.** 7-méthoxyméthyl-3-méthyl-1-(5-oxohexyl)xanthine.

**11.** 1-(5-hydroxyhexyl)-7-méthoxyméthyl-3-méthyl-xanthine racémique et ses énantiomères R en S.

**Revendications pour les Etats contractants suivants : ES, GR**

**1.** Procédé pour la préparation énantiosélective de S-(ω-1)-hydroxyalkylxanthines, caractérisé en ce que l'on met *à* incuber *avec Rhodocorula rubra* DSM 5436 un composé de formule générale I

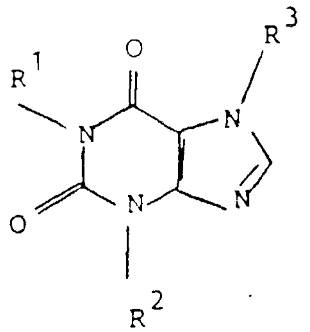

I

dans laquelle $R^1$, $R^2$ et $R^3$, qui peuvent être identiques ou différents, sont choisis dans le groupe constitué par les substituants $CH_3$-CO-$(CH_2)_m$- et alkyle en $C_1$-$C_6$, l'un des substituants devant être le groupe $CH_3$-CO-$(CH_2)_m$, m représentant un nombre entier allant de 2 à 6, et le groupe alkyle en $C_1$-$C_6$ pouvant être à chaîne droite ou ramifiée, ou dans laquelle
$R^1$ représente le groupe $CH_3$-CO-$(CH_2)_4$-, $R^2$ représente le groupe $CH_3$- et $R^3$ représente le groupe $CH_3$-O-$CH_2$-.

2. Procédé selon la revendication 1, caractérisé en ce que l'on utilise comme composé de formule générale I le composé dans lequel un seul des substituants $R^1$, $R^2$ et $R^3$ représente le groupe $CH_3$-CO-$(CH_2)_m$.

3. Procédé selon la revendication 1, caractérisé en ce que l'on utilise comme composé de formule générale I le composé dans lequel

a)

$R^1$ représente $CH_3$-CO-$(CH_2)_4$-,
$R^2$ représente $CH_3$- et
$R^3$ représente $CH_3$-$(CH_2)_2$- ou

b)

$R^1$ et $R^2$ représente $CH_3$- et
$R^3$ représente $CH_3$-CO-$(CH_2)_4$- ou

c)

$R^1$ représente $CH_3$-CO-$(CH_2)_4$-,
$R^2$ représente $CH_3$- et
$R^3$ représente $CH_3$-O-$CH_2$- ou

d)

$R^1$ représente $CH_3$-CO-$(CH_2)_4$- et
$R^2$ et $R^3$ représentent $CH_3$-, ou

e)

$R^1$ et $R^2$ représentent $CH_3$-$(CH_2)_3$- et
$R^3$ représente $CH_3CO(CH_2)_2$- ou

f)

$R^1$ représente $CH_3$-CO-$(CH_2)_3$-,
$R^2$ représente $CH_3$- et
$R^3$ représente $CH_3$-$(CH_2)_2$- ou

g)

$R^1$ représente $CH_3$-CO-$(CH_2)_5$- et
$R^2$ et $R^3$ représentent $CH_3$-.

**4.** Procédé selon une ou plusieurs des revendications 1 à 3, caractérisé en ce que l'on effectue l'incubation à 25-32°C.

**5.** Procédé pour la préparation énantiosélective de R-(-)-(ω-1)-hydroxyalkylxanthines, caractérisé en ce que, dans une première étape, on prépare une S-(+)-(ω-1)-hydroxyalkylxanthine, selon un procédé conforme à l'une des revendications 1 à 4, à la suite de quoi, dans une seconde étape, on fait réagir ladite S-(+)-(ω-1)-hydroxyalkylxanthine avec une phosphine tertiaire, un acide carboxylique et un azodicarboxylate de dialkyle, dans un solvant aprotique, et on convertit le produit de réaction, par solvolyse, en un composé de formule I selon la revendication 1, dans lequel l'un des substituants $R^1$, $R^2$ et $R^3$ représente le groupe R-(-)-$CH_3$-CH(OH)-$(CH_2)_m$.

**6.** Procédé selon la revendication 5, caractérisé en ce que la phosphine tertiaire est la triphénylphosphine, l'acide carboxylique est l'acide benzoïque, l'azodicarboxylate de dialkyle est l'azodicarboxylate de diéthyle, le solvant aprotique est le tétrahydrofuranne et la solvolyse est effectuée sous forme d'une méthanolyse en présence de carbonate de potassium.

**7.** Procédé pour la préparation énantiosélective de R-(-)-(ω-1)-hydroxyalkylxanthines, caractérisé en ce que, dans une première étape, on prépare une S-(+)-(ω-1)-hydroxyalkylxanthine, selon un procédé conforme à l'une des revendications 1 à 4, à la suite de quoi, dans une seconde étape

a) on fait réagir ladite S-(-)-(ω-1)-hydroxyalkylxanthine avec des halogénures de sulfonyle organiques, dans des solvants aprotiques,
b) on fait réagir le produit avec des sels alcalins d'acides carboxyliques aliphatiques, dans des solvants aprotiques, et
c) on convertit le produit par solvolyse dans des solvants aqueux ou alcooliques, en présence de substances basiques, en un composé de formule I selon la revendication 1, dans lequel l'un des substituants $R^1$, $R^2$ et $R^3$ représente le groupe R-(-)-$CH_3$-CH(OH)-$(CH_2)_m$.

**8.** procédé selon la revendication 7, caractérisé en ce que selon a) l'halogénure de sulfonyle organique est le chlorure de méthanesulfonyle ou le chlorure de p-toluènesulfonyle, le solvant aprotique est la pyridine ou le dichlorométhane, et en ce que selon b) le sel alcalin d'acides carboxyliques aliphatiques est le propionate de césium, le solvant aprotique est le diméthylformamide ou le diméthylsulfoxyde et en ce que selon c) le solvant est le méthanol et la substance basique est le carbonate de potassium.

**9.** *Rhodotorula rubra* DSM 5436 ainsi que ses mutants et variants, dans la mesure où le procédé selon la revendication 1 peut être mis en oeuvre avec ces derniers.